Europäisches Patentamt

European Patent Office

Office européen des brevets

(11) Veröffentlichungsnummer: **0 101 890**
**B1**

(12) # EUROPÄISCHE PATENTSCHRIFT

(45) Veröffentlichungstag der Patentschrift:
24.09.86

(51) Int. Cl.⁴: **A 61 M 1/34, A 61 M 25/00**

(21) Anmeldenummer: 83107131.1

(22) Anmeldetag: 21.07.83

(54) **Doppellumiger Katheter für eine Vorrichtung zur in-vivo-Reinigung von Blut.**

(30) Priorität: 30.07.82 DE 3228438

(43) Veröffentlichungstag der Anmeldung:
07.03.84 Patentblatt 84/10

(45) Bekanntmachung des Hinweises auf die Patenterteilung:
24.09.86 Patentblatt 86/39

(84) Benannte Vertragsstaaten:
BE CH DE FR GB IT LI SE

(56) Entgegenhaltungen:
EP-A-0 001 074
EP-A-0 025 704
WO-A-79/01121
DE-A-2 334 230
FR-A-2 201 906
FR-A-2 297 640
US-A-2 230 218
US-A-3 630 207
US-A-4 134 402

(73) Patentinhaber: Aigner, Karl, Dr., Uhlandstrasse 5, D-6301 Pohlheim (DE)

(72) Erfinder: Aigner, Karl, Dr., Uhlandstrasse 5, D-6301 Pohlheim (DE)

(74) Vertreter: Sternagel, Hans- Günther, Dr., Patentanwälte Dr. Michael Hann Dr. H.- G. Sternagel Sander Aue 30, D-5060 Bergisch Gladbach 2 (DE)

EP 0 101 890 B1

## Beschreibung

Gegenstand der Erfindung ist ein doppellumiger Katheter, der in Kombination mit einem Filter zur in-vivo-Reinigung von Blut verwendet werden kann, wobei aus einer Vene abgepumptes Blut einer intensiven Ultrafiltration unterzogen wird, und das Retentat zusammen mit einer dem Filtrat etwa volumenmäßig entsprechenden Menge Substitutflüssigkeit wieder der Vene zugeführt wird. Die Gesamtvorrichtung ist insbesondere dazu geeignet, im Rahmen intraarterieller Chemotherapie in hoher Konzentration eingebrachte Stoffe herauszufiltrieren, ehe das venöse Blut ins Herz und anschließend in den Körperkreislauf gelangt.

Die Ultrafiltration von menschlichem Blut bei Nierenversagen, um dadurch eine Nierenersatzfunktion zu erreichen, ist bekannt. Dabei wird Blut aus einer Arterie entnommen und unter dem Eigendruck durch ein Ultrafilter oder ein sogenanntes Hämofilter geleitet und das Retentat gegebenenfalls zusammen mit Substitutflüssigkeit in eine Vene zurückgeführt.

Im Rahmen intraarterieller Chemotherapie, insbesondere bei der Tumorbehandlung, ist es wünschenswert, die geeigneten Chemotherapeutika in möglichst hoher Konzentration an die gewünschte Behandlungsstelle heranzubringen. Die Toxizität und andere Nebenwirkungen limitierten bisher die Verträglichkeit und damit die maximal mögliche Dosierung.

Katheter für medizinische Zwecke sind seit langem bekannt und finden vielfach Anwendung.

In DE-A- 30 10 841 ist ein Doppelkatheter beschrieben, dessen Katheterrohre gegeneinander verschiebbar ausgebildet sind und der für die Venenpunktion zur Hämodialyse verwendet werden kann. Durch die Verschiebbarkeit der Einzelkatheter können die Eintrittsöffnungen bzw. Austrittsöffnung der Einzelkatheter an ihren Spitzen nach dem Einführen in ein Blutgefäß in einem solchen Abstand voneinander plaziert werden, daß in das Blutgefäß zurückgeführtes Blut nicht in den Bereich der Entnahmestelle gelangt.

Aus der EP-A- 0 025 704 ist ein doppellumiger Katheter bekannt, dessen Lumen koaxial gegeneinander verschiebbar ausgebildet sind, so daß nur ein Teil in das Blutgefäß eingebracht werden muß. Das innere Lumen kann ausgetauscht werden, ohne das äußere mit größerem Durchmesser aus dem Blutgefäß herausziehen zu müssen. Die Katheterspitze ist offen und es sind längs des Katheters noch mehrere seitliche Öffnungen vorhanden, die aufgrund der zwei Lumen gleichzeitig Blutentnahme und Rückführung ermöglichen.

Diese bekannten doppellumigen Katheter, deren Lumen gegeneinander verschiebbar ausgebildet sind, ermöglichen zwar die gleichzeitige Entnahme und Rückführung von Blutflüssigkeit in ein Gefäß zum Zwecke von Hämodialyse, sind jedoch für bestimmte intraarterielle Chemotherapien wenig geeignet oder sogar unbrauchbar, weil sich Entnahmestelle und Rückführungspunkt nicht ausreichend genau fixieren lassen.

Aus FR-A-2 297 640 ist ein Doppellumenkatheter mit koaxial angeordneten Katheterrohren bekannt, der die gleichzeitige Entnahme und Wiedereinführung von Flüssigkeit in ein Blutgefäß ermöglicht. Das äußere Katheterrohr hat zwischen Ansaugöffnung und den seitlichen Rückfuhröffnungen in der Nähe des geschlossenen Katheterendes einen elastischen Ballon, der sich im aufgeblasenen Zustand an die Gefäßinnenwand anlegt und den Durchtritt durch das Gefäß sperrt, so daß Entnahme- und Rückfuhrstelle räumlich voneinander getrennt sind und das gesamte venöse Blut einer externen Reinigung zugeführt wird. Wird ein solcher Katheter in der Hauptvene bis in die Nähe des Herzens eingeführt, treten durch den vollständigen Abschluß des Gefäßes erhebliche Störungen im Strömungsprofil auf, die sich negativ auf den Herzrhythmus auswirken, so daß eine solche Anordnung des Katheters in der Hauptvene nicht möglich ist.

Aufgabe der vorliegenden Erfindung ist es, einen doppellumigen Katheter zu schaffen, der eine in-vivo-Reinigung von Blut im Rahmen einer intraarteriellen Chemotherapie ermöglicht, wobei eingebrachte hohe Mengen therapeutisch wirksamer Substanzen mittels des Katheters nach der Behandlungsstelle mit dem Blut entnommen und aus dem venösen Blut herausfiltriert werden, um dadurch toxische Wirkungen und Nebenreaktionen hinter der Behandlungsstelle zu vermeiden, ohne daß der Durchfluß durch das Gefäß durch den eingebrachten Katheter gesperrt wird und der einen Rückfluß mit möglichst geringen Störungen der Strömung ermöglicht.

Diese Aufgabe wird gelöst durch einen doppellumigen Katheter, dessen erstes Katheterteil eine geschlossene Spitze und mehrere seitlich in der Nähe der Spitze angeordnete Öffnungen aufweist und mit einem im ersten Katheterteil angeordneten zweiten Katheterteil das mit zwei oder mehr Öffnungen in der Außenwand des ersten Katheterteils endet, wobei die Öffnungen am Ende des zweiten Katheterteils so groß sind wie der Querschnitt des zweiten Katheterteils, der dadurch gekennzeichnet ist, daß der Abstand zwischen der von der Katheterspitze am weitesten entfernten seitlichen Öffnung des ersten Katheterteils und der nächstliegenden Öffnung des zweiten Katheterteils nicht weniger als 40 mm und nicht mehr als 50 mm beträgt, und daß zwischen den Öffnungen der Außen des ersten Katheterteils auf dessen Aussenseite ein etwa 2 mm dicker Wulst, der sich über den halben Außenumfang erstreckt, vorhanden ist.

In den abhängigen Ansprüchen sind bevorzugte Ausführungsformen der Erfindung beschrieben.

Der doppellumige Katheter ist Teil einer Vorrichtung zur in-vivo-Reinigung von Blut, wobei er in Kombination mit jeweils an die Lumen anschließenden Schlauchverbindungen verwendet wird, und der Schlauch vom zweiten Katheterteil über eine zwischengeschaltete erste Schlauchpumpe mit der Eingangsseite eines Ultrafiltrationsfilters verbunden ist und der Schlauch die Ausgangsseite des Ultrafiltrationsfilters für die Rückführung des Retentats mit dem ersten Kathetarteil verbindet, in den Schlauch hinter dem Ultrafiltrationsfilter eine Zuführleitung mit einer dazwischengeschalteten zweiten Schlauchpumpe für Substitutflüssigkeit mündet, in den Schlauch vor dem Ultrafiltrationsfilter eine Zuführleitung für Antikoagulantien mündet und die Abgangsleitung für Filtrat aus dem Ultrafiltrationsfilter über ein genau einzustellendes Ventil in einem Meßgerät endet.

Um die Blutentnahme aus einer Körpervene zu ermöglichen, wird ein doppellumiger Katheter mit einer geschlossenen Spitze eingesetzt. Der Katheter weist ein erstes Katheterteil mit mehreren seitlich in der Nähe der Spitze angeordneten Öffnungen und innerhalb des ersten Katheterteils ein zweites Katheterteil auf, das mit zwei oder mehreren Öffnungen in der Außenwand des Katheters endet, wobei der Abstand zwischen der von der Katheterspitze am weitesten entfernten seitlichen Öffnung im Endbereich des Katheters und der nächstliegenden Öffnung des zweiten Katheterteils nicht weniger als 40 mm und nicht mehr als 50 mm beträgt.

Der Katheter ist als ein Doppelschlauch ausgebildet, wobei der erste Katheterteil B als Rückflußschlauch dient und der zweite Katheterteil A als Absaugschlauch. Der Querschnitt der beiden Katheterteile kann gleich groß oder unterschiedlich ausgebildet sein. Bei unterschiedlicher Querschnittsgröße ist der Querschnitt des zweiten Katheterteils A kleiner als der des Teils B. Vorteilhafterweise wird der Katheter von der Leiste aus in der Hauptkörpervene hochgeschoben bis die Katheterspitze im rechten Vorhof des Herzens liegt. Um strömungsbedingte Störungen, die zu Herzrhythmusstörungen führen können, bei der Rückführung des Blutes auszuschließen, ist die eigentliche Katheterspitze geschlossen, und das rückgeführte Retentat tritt aus mehreren seitlichen Öffnungen aus.

Wesentlich ist, daß die Absaugstelle nicht weniger als 40 mm und nicht mehr als 50 mm von der nächstgelegenen Zuführöffnung entfernt ist.

Das venöse Blut wird mit einer Schlauchpumpe oder sogenannten Rollerpumpe abgesaugt und mit dem erhöhten Pumpendruck durch ein übliches Ultrafiltrationsfilter gepreßt und das Retentat in einer Menge von 400-500 ml pro Minute dem ersten Katheterteil zugeführt. Da bei der Filtration gleichzeitig 100-200 ml pro Minute Filtratflüssigkeit aus dem Ultrafiltrationsfilter austreten, ist die Zuführung einer entsprechenden Menge an Substitutflüssigkeit zum Ausgleich erforderlich; um dies zu ermöglichen, weist die Schlauchverbindung zwischen dem Ultrafiltrationsfilterausgang für das Retentat und den Katheter einen Anschluß für Substitutflüssigkeit auf, wobei in der Anschlußleitung eine zweite Schlauchpumpe angeordnet ist, um die benötigte Menge an Substitutflüssigkeit in das System einspeisen zu können. Der Filtratabgang wird über ein genau einstellbares Ventil geregelt, und die Abführleitung endet in einem Auffanggefäß mit Meßmöglichkeit, um die dem Kreislauf entzogene Flüssigkeitsmenge volumenmäßig zu bestimmen.

Zwischen der saugenden Schlauchpumpe und dem Katheter mündet in der Schlauchleitung eine Zuführleitung für Antikoagulantien, z.B. Heparin. Diese etwa 1 m lange Zuführleitung weist einen Innendurchmesser von 1,5 mm und einen Außendurchmesser von 3 mm auf und ist an ihrem anderen Ende an einen genau einstellbaren Injektionsautomaten angeschlossen.

Das Verhältnis der freien Querschnitte des ersten Katheterteils B und des zweiten Katheterteils A beträgt 1:1 bis 2:1. Der Innendurchmesser des Katheterschlauches liegt nicht unter 3 mm und beträgt vorzugsweise 4 mm, so daß sich ein Außendurchmesser von etwa 4-5 mm für den Katheter ergibt.

Die Ausflußöffnungen des ersten Katheterteils B sind am Katheterende auf einer Strecke von etwa 15-20 mm angeordnet, und die Öffnungen haben einen Durchmesser von 1,5-4 mm. Die Öffnung des zweiten Katheterteils A oder Absaugschlauches, der im inneren des Katheters angeordnet ist, sind vorzugsweise so groß wie der Querschnitt dieses Schlauches und sind in dem bereits angegebenen Abstand vom letzten Zuführloch in der Außenwand des Katheters angeordnet. Das zweite Katheterteil endet an dieser Stelle des Katheters. Vorzugsweise sind zwei Ansaugöffnungen vorgesehen, zwischen denen auf der Außenseite der Katheterwand ein etwa 2 mm dicker Wulst, der sich über den halben Außenumfang des Katheters erstreckt, also ein Halbringwulst ist, vorhanden sein kann, um sicherzustellen, daß die Ansaugöffnungen nicht durch die Gefäßwand abgedeckt und verschlossen werden. Der doppellumige Katheter ist ca. 60-80 cm lang und weist vorzugsweise im Abstand von 45 cm vom Katheterende eine Markierung auf, um die eingeführte Länge besser kontrollieren zu können. Am rückwärtigen Ende des Katheters werden die zwei Katheterteile voneinander getrennt, wobei das erste Teil ein etwa 20 cm langes und das zweite Teil ein etwa 10 cm langes Anschlußstück aufweisen.

Die Verbindung vom Katheter zur Schlauchpumpe bzw. Ultrafiltrationsfilter erfolgt über Schlauchverbindungen, wobei vor und hinter der ersten Schlauchpumpe, die das Blut ansaugt und in das Filter drückt, jeweils eine eingefaßte Kautschukmembran in die Schlauchverbindung eingesetzt wird, um Injektionen oder Entnahme von Kontrollproben mit Injektionsspritzen zu ermöglichen. Weitere solche Membranen können in die Filtratleitung nach dem Ultrafiltrationsfilter vor dem Ventil und in der Schlauchverbindung für das Retentat zum Katheter vorhanden sein. Um den Druck und die Füllung im System besser kontrollieren zu können, kann zwischen der Schlauchpumpe und dem Ultrafiltrationsfilter ein Schlauchstück aus elastischem Material in Kissenform angeordnet sein, das sich unter dem von der Schlauchpumpe erzeugten Druck aufweitet und dessen Volumenvergrößerung oder Verkleinerung ein Maß für den durch die Pumpe erzeugten Druck und den Füllungszustand im System ist.

Von besonderem Vorteil für die Beobachtung des Füllungszustandes ist es, wenn in der Schlauchleitung für das Retentat zum Katheter ein weiteres solches Schlauchstück in Kissenform angeordnet ist.

Die Kissen wirken auch als Luftblasenfalle.

Um Wärmeverluste durch Abkühlung innerhalb der Vorrichtung ausgleichen zu können, ist bei einer bevorzugten Ausführungsform vorgesehen, in die Rückführschlauchleitung eine etwa 3-7 m, vorzugsweise 5 m, lange Schlauchspirale, die in einem auf 40°C temperierten Wasserbad angeordnet ist, zwischenzuschalten. Dies ermöglicht eine Aufwärmung des filtrierten Blutes und der zugeführten Substitutflüssigkeit auf die gewünschte Körpertemperatur. Der Katheter kann aus den für Kathetermaterialien üblichen und geeigneten Materialien hergestellt werden. Derartige Materialien verhalten sich neutral gegenüber der Körperflüssigkeit, lassen sich ohne Probleme sterilisieren und sind ausreichend elastisch, aber andererseits auch genügend steif und fest, um in Blutgefäße eingeführt zu werden. Geeignete Materialien sind Polyolefine, polyfluorierte Kohlenwasserstoffpolymere, synthetische Kautschuke, Polyvinylchlorid und dergleichen. Besonders bevorzugte Materialien für den Katheter sind Silikonkautschuk und implantierbares Polyvinylchlorid. Für die Schlauchverbindungen sind grundsätzlich vergleichbare Materialien geeignet, insbesondere werden jedoch Polyolefine, fluorierte Kohlenwasserstoffpolymere oder Polyvinylchlorid für die Schläuche verwendet. Bei den Schlauchpumpen handelt es sich um handelsübliche und bekannte Schlauchpumpen oder sogenannte Rollerpumpen, bei denen die Pumpwirkung durch Zusammendrücken des Schlauches erzeugt wird.

Als Ultrafiltrationsfilter sind übliche Ultrafiltrationsfilter mit den üblichen Membranen geeignet, sofern die Filterfläche und Filterleistung ausreichend ist, um die erforderliche Menge an niedermolekularen Produkten aus dem Blut sicher zu entfernen. Die erforderliche Filterfläche liegt in der Größenordnung von 1-2,5 m², vorzugsweise 1,4-2,0 m²; ganz besonders bevorzugt ist eine Filterfläche von 2 m². Geeignete Membranen sind solche, die Substanzen bis zu einem Molekulargewicht von etwa 40 000 bis 60 000 durchlassen, gegenüber höher molekularen Stoffen jedoch sperren. In besonders gelagerten Fällen können auch andere Membranen eingesetzt werden, die nur niedermolekulare Produkte bis zu einer Molekülgröße von etwa 20 000 durchlassen.

Die erfindungsgemäße Vorrichtung ist insbesondere zur Verwendung für die Cytostatika-Filtration bei intraarterieller Chemotherapie einsetzbar und ermöglicht eine systemische Behandlung mit lokal hoher Konzentration, wobei nach der Einwirkung die Cytostatika und die anderen therapeutisch wirksamen Substanzen aus dem Blut mittels des Ultrafilters herausfiltriert werden und damit toxische Nebenwirkungen vermieden werden, die insbesondere dann auftreten können, wenn diese Stoffe mit dem Blut bis zum Herzen und dann in den Körperkreislauf gelangen.

Der Gegenstand der Erfindung wird nun anhand der Abbildungen noch näher beschrieben.

Abbildung 1 zeigt eine schematische Gesamtübersicht der erfindungsgemäßen Vorrichtung.

Abbildung 2 und 2a zeigen die Ausbildung des Katheters.

Abbildung 3 und 3a zeigen den Katheter im Querschnitt und lassen die Anordnung des zweiten Katheterteils im ersten Katheterteil erkennen.

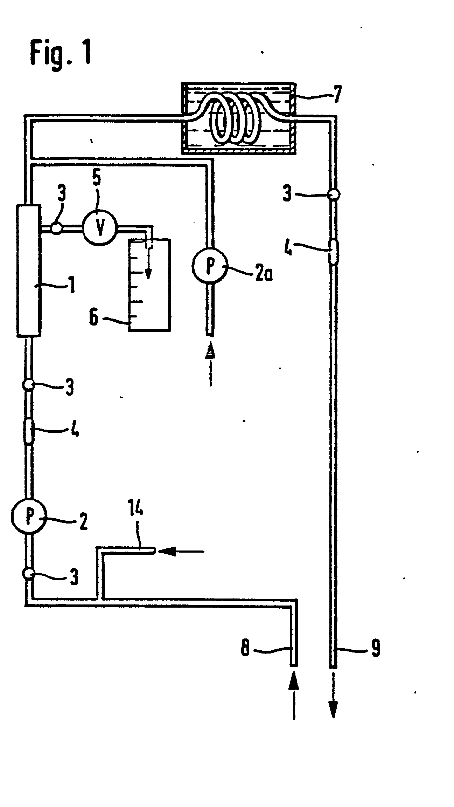

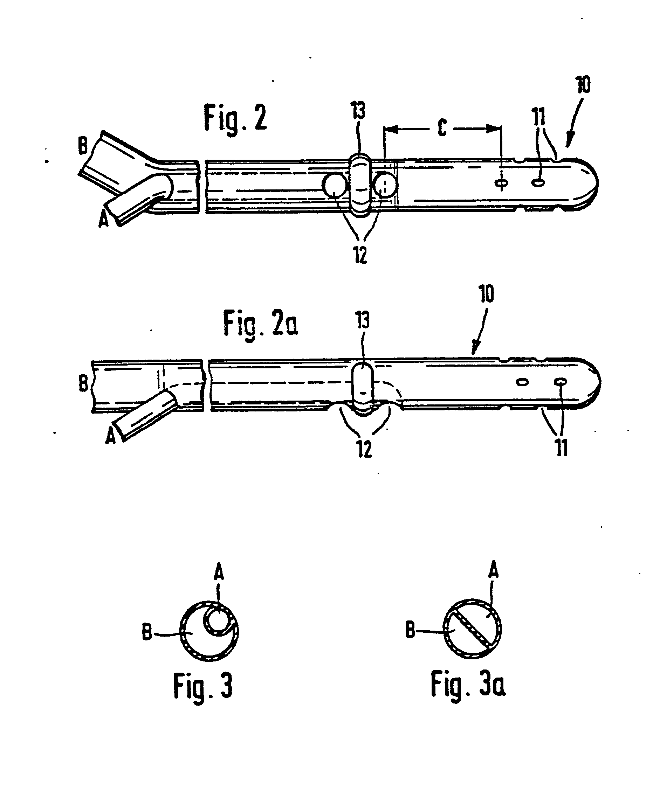

Figur 1 zeigt in schematischer Übersicht die erfindungsgemäße Vorrichtung, wobei an die Schlauchverbindungen 8, 9 der in Figur 2 gezeigte doppellumige Katheter derart angeschlossen, daß die Absaugleitung an den Schlauch 8 und die Rückführleitung an den Schlauch 9 angeschlossen ist. Aus dem zweiten Katheterteil A wird venöses Blut über die Schlauchverbindung 8 von der Schlauchpumpe 2 angesaugt und durch den anschließenden Schlauch in das Ultrafiltrationsfilter 1 gedrückt und gelangt von diesem durch die Schlauchverbindung 9 zum ersten Katheterteil B. In die Schlauchverbindung 8 mündet vor dem Filter 1 die Zuführleitung 14 für Koagulatverhinderungsmittel, die von einer automatischen Injektionsapparatur kommt. In die Schlauchverbindung 8 sind vor und hinter der Schlauchpumpe 2 jeweils eingefaßte Kautschukmembranen 3 eingeschaltet, um Injektionen vornehmen zu können, bzw. Kontrollproben mit Injektionsspritzen abziehen zu können. Die gleichen Kautschukmembranen sind in der Filtratleitung zwischen dem Filter 1 und dem Ventil 5 und in der Schlauchleitung 9 hinter der im Wasserbad angeordneten Spirale 7 vorhanden.

Mit 4 sind die elastischen Kautschukkissen zur Füllungskontrolle bezeichnet. Vom Ultrafilter 1 geht die Filtratleitung ab, in der das genau einstellbare Ventil 5 angeordnet ist und die in einem Meßgefäß 6 zum Auffangen des Filtrats endet. Das Meßgefäß weist eine Anzeigeskala auf und hat ein Fassungsvermögen von 1-3 Liter. Hinter dem Ultrafiltrationsfilter 1 befindet sich in der Schlauchverbindung 9 die Zuführstelle für Substitutflüssigkeit, die von einer zweiten Schlauchpumpe 2a eingebracht wird. Die Schlauchpumpe 2a saugt die Substitutflüssigkeit aus ein oder mehreren über Schlauchverbindungen angeschlossenen Vorratsgefäßen ausreichender Größe an. Gegebenenfalls sind in der Schlauchverbindung Verzweigungen mittels T-Stücken vorhanden, um mehrere unterschiedliche Substitutflüssigkeiten verwenden zu können. Das Einpumpen der Substitutflüssigkeit, für die vorzugsweise sogenannte Ringer-Lösungen verwendet werden, ist erforderlich, weil sich sonst die großen Mengen an Substitutflüssigkeit nicht mit der gewünschten Genauigkeit und Geschwindigkeit in das System einbringen lassen. Die Leistung der zweiten Schlauchpumpe 2a wird auf die durch das einstellbare Ventil 5 aus dem System austretende Flüssigkeitsmenge derart eingeregelt, daß volumenmäßig ein Flüssigkeitsverlust möglichst vermieden wird. Grundsätzlich ist es jedoch auch möglich, geringere oder höhere Mengen an Substitutflüssigkeiten einzubringen, wenn dies in einem speziellen Fall in Rahmen der Gesamttherapie erforderlich ist.

Um Abkühlungsverluste auszugleichen, ist vorzugsweise in der Schlauchleitung 9 eine Schlauchspirale 7 angeordnet, die sich in einem Wasserbad befindet, das auf etwa 40°C temperiert ist. Auf diese Weise können auftretende Wärmeverluste in einfacher Weise kompensiert werden, so daß das Retentat nach der Filtration mit gewünschter Temperatur in die Vene zurückgeführt werden kann. Die Schlauchverbindung zwischen dem Katheter und der Schlauchpumpe hat etwa eine Länge von 1,5 mm, der Schlauchabstand zwischen Schlauchpumpe und Filter beträgt etwa 1 m, und etwa 2 m Schlauchverbindungen ohne die Schlauchspirale sind zum Anschluß des Schlauches 9 an den Katheter erforderlich. Die Schlauchverbindungen haben einen Innendurchmesser von etwa 5 mm und einen Außendurchmesser von etwa 7 mm.

Figur 2 zeigt den doppellumigen Katheter 10 von der Seite mit dem geschlossenen Ende des ersten Katheterteils B und den in der Nähe angeordneten seitlichen Öffnungen 11. Im Inneren des ersten Katheterteils B ist das zweite Katheterteil A angeordnet, das mit den Öffnungen 12 in der Katheterwand endet. Der Abstand C zwischen der als Ansaugöffnung dienenden Öffnung 12 und der nächstgelegenen Rückführöffnung 11 im ersten Katheterteil B beträgt nicht weniger als 40 mm und nicht mehr als 50 mm. Zwischen zwei Absaugöffnungen 12 ist auf der Außenseite der Katheterwand ein Halbringwulst 13 bei einer bevorzugten Ausführungsform angeordnet, um ein anliegen der Gefäßwand an den Katheter und ein Verschließen der Ansaugöffnungen zu vermeiden.

Figur 2a zeigt den Katheter in Aufsicht mit den seitlichen Ansaugöffnungen 12 und den in Abstand an der Katheterspitze angeordneten Rückführöffnungen 11 des ersten Katheterteils B.

Figur 3 und 3a zeigen die möglichen Ausbildungsformen der ineinander angeordneten Katheterteilen A und B, wobei vorzugsweise die Ausführungsform von Figur 3 gewählt wird, bei der in einem Schlauch B ein zweiter Schlauch A mit geringerem Querschnitt angeordnet ist. Das Verhältnis der Querschnittsflächen B:A beträgt vorzugsweise 1:1, d.h. der Gesamtquerschnitt des Katheters weist die doppelte Fläche des zweiten Katheterteils A auf. Bei der Ausbildung nach Figur 3a weist der Katheter im Inneren eine Trennwand auf, um die Katheterteile A und B voneinander abzutrennen.

**Patentansprüche**

1. Doppellumiger Katheter, dessen erstes Katheterteil (B) eine geschlossene Spitze und mehrere seitlich in der Nähe der Spitze angeordnete Öffnungen (11) aufweist und mit einem im ersten Katheterteil (B) angeordneten zweiten Katheterteil (A), das mit zwei oder mehr Öffnungen (12) in der Außenwand des ersten Katheterteils (B) endet, wobei die Öffnungen (12) am Ende des zweiten Katheterteils (A) so groß sind wie der Querschnitt des zweiten Katheterteils (A),

dadurch gekennzeichnet,

daß der Abstand zwischen der von der Katheterspitze am weitesten entfernten seitlichen Öffnung (11) des ersten Katheterteils (B) und der nächstliegenden Öffnung (12) des zweiten Katheterteils (A) nicht weniger als 40 mm und nicht mehr als 50 mm beträgt, und daß zwischen den Öffnungen (12) in der Außenwand des ersten Katheterteils (B) auf dessen Außenseite ein etwa 2 mm dicker Wulst (13), der sich über den halben Außenumfang erstreckt, vorhanden ist.

2. Doppellumiger Katheter nach Anspruch 1, dadurch gekennzeichnet, daß das Verhältnis der Querschnitte des ersten und des zweiten Katheterteils 1:1 bis 2:1 beträgt.

3. Doppellumiger Katheter nach Ansprüchen 1 oder 2, dadurch gekennzeichnet, daß der Katheter im Inneren eine Trennwand aufweist, die die Katheterteile (A, B) voneinander trennt.

4. Vorrichtung zur in-vivo-Reinigung von Blut mit einem doppellumigen Katheter nach Ansprüchen 1 bis 3 in Kombination mit jeweils an die Lumen anschließenden Schlauchverbindungen (8, 9), wobei der Schlauch (8) vom zweiten Katheterteil (A) über eine zwischengeschaltete erste Schlauchpumpe (2) mit der Eingangsseite eines Ultrafiltrationsfilters (1) verbunden ist und der Schlauch (9) die Ausgangsseite des Ultrafiltrationsfilters, (1) für die Rückführung des Retentats mit dem ersten Katheterteil (B) verbindet, in den Schlauch (9) hinter dem Ultrafiltrationsfilter (1) eine Zuführleitung mit einer dazwischengeschalteten zweiten Schlauchpumpe (2a) für Substitutflüssigkeit mündet, in den Schlauch (8) vor dem Ultrafiltrationsfilter (1) eine Zuführleitung für Antikoagulantien mündet und die Abgangsleitung für Filtrat aus dem Ultrafiltrationsfilter (1) über ein genau einzustellendes Ventil (5) in einem Meßgerät (6) endet.

## Claims

1. A double-lumen catheter of which the first part (B) has a closed tip and a plurality of openings (11) which are laterally positioned in the vicinity of the tip having a second catheter part (A) which is located within the first catheter part (B) whereby this second part terminates in two or more openings (12) in the outer wall of the catheter part (B), and the openings (12) at the end of the second catheter part (A) are as large as the cross section of the second catheter part (A),
   characterized in that,
   the distance between the farthest lateral opening (11) from the tip of the first catheter part (B) and the nearest opening (12) of the second catheter part (A) thereto is not less than 40 mm and not more than 50 mm and that between the openings (12) in the outer wall a bulge (13) about 2 mm thick extends over half of the outer circumference of the outer side of the first catheter part (B).

2. The double-lumen catheter of claim 1,
   characterized in that,
   the ratio of the cross sectional areas of the first and the second catheter parts is from 1:1 to 2:1.

3. The double-lumen catheter of claims 1 or 2,
   characterized in that,
   the catheter has a separating wall within its interior, which separates the catheter parts (A) and (B) from each other.

4. A device for the in-vivo purification of blood having a double-lumen catheter according to claims 1 to 3, each lumen having a tube connection (8, 9) whereby the tube (8) from the second catheter part (A) is connected via a first intermediate tube pump (2) to the inlet side of the ultrafiltration filter (1), and the tube from (9) connects the outlet side of the ultrafiltration filter (1), for the return of the filtrate, to the first catheter part (B), whereby a feed line for substitute fluid via a second intermediate tube pump (2a) opens into the tube (9) behind the ultrafiltration filter (1), and a feed line for anticoagulants opens into the tube (8) in front of the ultrafiltration filter (1), and the outflow line for the filtrate from the ultrafiltration filter (1) passes through a precisely adjustable valve (5) and terminates in a measuring device (6).

## Revendications

1. Cathéter à double lumière, dont la première partie de cathéter (B) présente une pointe fermée et plusieurs orifices (11) disposés latéralement au voisinage de la pointe et qui présente une seconde partie de cathéter (A) disposée dans la première partie de cathéter (B) et se terminant par deux orifices (12) ou plus situés dans la paroi extérieure de la première partie de cathéter (B), ces orifices (12) situés à l'extrémité de la seconde partie de cathéter (12) étant aussi grands que la section transversale de cette seconde partie de cathéter (A), caractérisé en ce que la distance entre l'orifice latéral (11) de la première partie de cathéter (B) le plus éloigné de la pointe du cathéter, et l'orifice (12), le plus proche, de la seconde partie de cathéter (A) ne s'élève pas à moins de 40 mm et à plus de 50 mm, et en ce qu'il est prévu entre les orifices (12) situés dans la paroi extérieure de la première partie de cathéter (B) et sur le côté extérieur de celle-ci, un bourrelet (13) épais d'environ 2 mm et qui s'étend sur la moitié de la périphérie extérieure.

2. Cathéter à double lumière selon la revendication 1, caractérisé en ce que le rapport des sections transversales de la première et de la seconde parties de cathéter est de 1:1 à 2:1.

3. Cathéter à double lumière selon les revendications 1 ou 2, caractérisé en ce que le cathéter comporte en son intérieur une paroi de séparation qui sépare l'une de l'autre les parties de cathéter (A, B).

4. Dispositif pour l'épuration du sang in vivo, présentant un cathéter à double lumière selon les revendications 1 à 3, en combinaison avec des liaisons à tuyau souple (8, 9) se raccordant à chaque fois à la lumière associée, dans lequel le tuyau souple (8) provenant de la deuxième partie de cathéter (A) est relié, par l'intermédiaire d'une première pompe tubulaire (2) intercalée, au côté d'entrée d'un filtre d'ultrafiltration (1), et le tuyau souple (9) relie le côté de sortie de ce filtre d'ultrafiltration (1), en vue de la réinjection du rétentat, à la première partie de cathéter (B), tandis que débouche dans ce tuyau souple (9), en aval du filtre d'ultrafiltration (1), une conduite d'amenée munie d'une seconde pompe tubulaire (2a) intercalée, prévue pour un fluide de substitution, et que débouche dans le tuyau souple (8), en amont du filtre d'ultrafiltration (1), une conduite d'amenée pour des anticoagulants, la conduite de sortie de filtrat hors du filtre d'ultrafiltration (1) se terminant, par l'intermédiaire d'une vanne (5) se réglant de manière précise, dans un appareil de mesure (6).

Fig. 1

Fig. 2

Fig. 2a

Fig. 3

Fig. 3a